# EUROPEAN PATENT APPLICATION

(11) **EP 4 108 241 A1**
(43) Date of publication of application: **28.12.2022**
(21) Application number: 21757364.1
(22) Date of filing: 09.02.2021
(51) Int. Cl.: A61K 31/42, A61K 31/277, A61P 31/14, A61P 31/16, A61P 31/18

(54) **ANTI-RNA VIRUS DRUG AND APPLICATION THEREOF**

(30) Priority: 18.02.2020 CN 202010099375
(71) Applicant: East China University of Science and Technology, Shanghai 200237 (CN); Wuhan University, Hubei 430072 (CN)
(72) Inventor: LI, Honglin, Shanghai 200237 (CN); XU, Ke, Wuhan, Hubei 430072 (CN); ZHAO, Zhenjiang, Shanghai 200237 (CN)
(74) Representative: reuteler & cie SA
(86) International application number: PCT/CN2021/076396
(87) International publication number: WO 2021/164672

(57) **Abstract**

An application of a compound represented by formula I or a pharmaceutically acceptable salt thereof in the preparation of a drug for treating RNA virus infection.

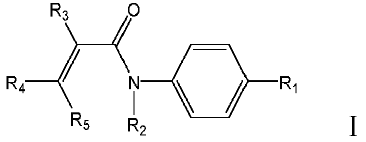

## Description

### Technical field

The invention relates to the field of medicinal chemistry; and in particular, the invention relates to the use of leflunomide, teriflunomide and the like in the preparation of a medicament for treating RNA virus infection and in the treatment of virus infection.

### Background

Diseases caused by viral infections are an important threat to public health security. The viruses include not only the well-known influenza virus, Ebola virus, bunya virus, avian influenza virus H9N2, H1N1, H7N9, arena virus, rabies virus, HCV, HBV, HIV-1, HSV-1, HSV-2, etc., but also coronaviruses, such as severe acute respiratory syndrome coronavirus (SARS-CoV), Middle East respiratory syndrome coronavirus (MERS-CoV) and 2019 novel coronavirus (2019-nCoV).

Coronaviruses are a large family that exists widely in nature, and are susceptible to humans and a variety of animals. They are named for the corona-like fibers on the surface of virus particles. Coronaviruses belong to the Coronaviridae. Based on the systematic analysis of viral nucleic acid sequences, the 9^{th} report of the International Committee on Taxonomy of Viruses classified coronaviruses into four categories: α, β, γ, and a new putative genus. Among them, 7 β coronaviruses can infect humans, namely human coronavirus 229E (HCoV-229E), human coronavirus NL63 (HCoV-NL63), human coronavirus OC43 (HCoV-OC43), Hong Kong type I human Coronavirus (HCoV-HKU1), Severe Acute Respiratory Syndrome Coronavirus (SARS-CoV), Middle East Respiratory Syndrome Coronavirus (MERS-CoV) and 2019 Novel Coronavirus (2019-nCoV). Among them, 2019-nCoV is currently breaking out, and causing serious harm to human health.

All of the diseases caused by acute viral infections share some common characteristics: 1) short course (1-2 weeks) and rapid development (rapid development within a few days after onset); 2) severe illness and even death are easily caused in high-risk groups; 3) it is easy to cause population spread; and 4) the rapid replication of the virus usually causes excessive inflammatory response.

At present, antiviral drugs mainly target functional proteins of viruses; that is, targeted drugs need to be developed for each virus. Such antiviral drug can achieve high specificity and selectivity, however, long-term and large-scale use often leads to drug resistance, and the research and development costs are long and lack predictability.

Viruses, as parasitic living organisms, must rely on the resources of host cells for reproduction. Therefore, there is an urgent need in the art for small molecule drugs designed for the host cells on which the virus lives, so that broad-spectrum antiviral drugs can be obtained.

### Summary of the invention

The purpose of the present invention is to provide drugs with broad-spectrum and excellent antiviral activities. Such drugs have low toxicities to normal cells, thereby laying a material basis for the research and development of a new generation of antiviral drugs.

In the first aspect, the present invention provides the use of a compound of formula I, or a pharmaceutically acceptable salt thereof, in the preparation of a drug against RNA virus infection: 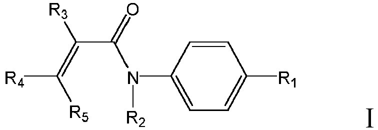 wherein, R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.

In a specific embodiment, in formula I,
R₁ is selected from: H, a substituted or unsubstituted C1-3 alkyl, substituted or unsubstituted C1-3 alkoxy;
R₂ is selected from: H, a substituted or unsubstituted C1-3 alkyl;
R₃ is selected from: H, a cyano, substituted or unsubstituted C1-3 alkyl, substituted or unsubstituted C1-3 alkoxy;
R₄ is selected from: H, a hydroxyl, substituted or unsubstituted C1-3 alkyl, substituted or unsubstituted C1-3 alkoxy; alternatively, R₃ and R₄ may be joined to form a 5-6 membered ring containing 2 heteroatoms selected from N, O or S;
R₅ is selected from: H, a substituted or unsubstituted C1-3 alkyl, substituted or unsubstituted C1-3 alkoxy.

In a preferred embodiment, R₁ is a trifluoromethyl; R₂ is H; and R₅ is a methyl.

In a specific embodiment, the compound of formula I is a compound selected from the group consisting of: 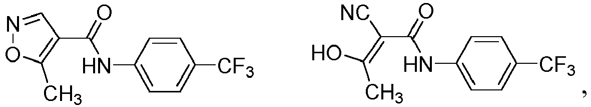 preferably, the compound of formula I is

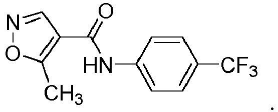

In specific embodiments, the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like;
preferably, the RNA virus is 2019 novel coronavirus (2019-nCoV), Ebola virus, avian influenza H9N2, H1N1 or H7N9.

In a preferred embodiment, the RNA virus includes, but not limited to: severe acute respiratory syndrome coronavirus (SARS-CoV), Middle East respiratory syndrome coronavirus (MERS-CoV) and 2019 novel coronavirus (2019-nCoV), Ebola virus, fever with thrombocytopenia syndrome virus (sftsv), avian influenza virus H9N2.

In the second aspect, the present invention provides a pharmaceutical composition comprising a compound of formula I, or a pharmaceutically acceptable salt thereof and other antiviral drugs, 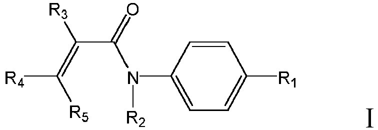 wherein R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.
In a preferred embodiment, in formula I,
R₁ is selected from: H, a substituted or unsubstituted C1-3 alkyl, substituted or unsubstituted C1-3 alkoxy;
R₂ is selected from: H, a substituted or unsubstituted C1-3 alkyl;
R₃ is selected from: H, a cyano, substituted or unsubstituted C1-3 alkyl, substituted or unsubstituted C1-3 alkoxy;
R₄ is selected from: H, a hydroxyl, substituted or unsubstituted C1-3 alkyl, substituted or unsubstituted C1-3 alkoxy; alternatively, R₃ and R₄ may be joined to form a 5-6 membered ring containing 2 heteroatoms selected from N, O or S; and
R₅ is selected from: H, a substituted or unsubstituted C1-3 alkyl, substituted or unsubstituted C1-3 alkoxy.

In a preferred embodiment, R₁ is a trifluoromethyl; R₂ is H; and R₅ is a methyl.

In a specific embodiment, the compound of formula I is a compound selected from the group consisting of: 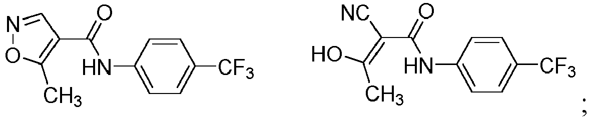 preferably, the compound of formula I is

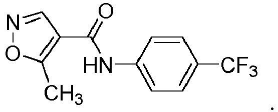

In a preferred embodiment, the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like;
preferably, the RNA virus is 2019 novel coronavirus (2019-nCoV), Ebola virus, avian influenza H9N2, H1N1 or H7N9.

In a preferred embodiment, the RNA virus includes, but not limited to: severe acute respiratory syndrome coronavirus (SARS-CoV), Middle East respiratory syndrome coronavirus (MERS-CoV) and 2019 novel coronavirus (2019-nCoV), Ebola virus, fever with thrombocytopenia syndrome virus (sftsv), avian influenza virus H9N2.

In a specific embodiment, the other antiviral drugs include, but not limited to: one or more of lopinavir, ritonavir, ribavirin, remdesivir, oseltamivir, Tamiflu, lanimil, weperamivir, arbidol and chloroquine (chloroquine phosphate) and the like; and preferably one or more of lopinavir, ritonavir, ribavirin, remdesivir and chloroquine (chloroquine phosphate).

In a specific embodiment, the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like.

In the third aspect, the present invention provides a method for treating RNA virus infection, comprising administering a therapeutically effective amount of a compound of formula I or a pharmaceutically acceptable salt thereof or the pharmaceutical composition described in the second aspect to a subject in need of the treatment for viral infections: 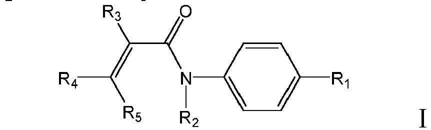 wherein R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.

In a preferred embodiment, in formula I,
R₁ is selected from: H, a substituted or unsubstituted C1-3 alkyl, substituted or unsubstituted C1-3 alkoxy;
R₂ is selected from: H, a substituted or unsubstituted C1-3 alkyl;
R₃ is selected from: H, a cyano, substituted or unsubstituted C1-3 alkyl, substituted or unsubstituted C1-3 alkoxy;
R₄ is selected from: H, a hydroxyl, substituted or unsubstituted C1-3 alkyl, substituted or unsubstituted C1-3 alkoxy; alternatively, R₃ and R₄ may be joined to form a 5-6 membered ring containing 2 heteroatoms selected from N, O or S; and
R₅ is selected from: H, a substituted or unsubstituted C1-3 alkyl, substituted or unsubstituted C1-3 alkoxy.

In a preferred embodiment, R₁ is a trifluoromethyl; R₂ is H; and R₅ is a methyl.

In a specific embodiment, the compound of formula I is a compound selected from the group consisting of: 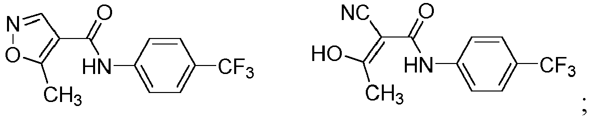 preferably, the compound of formula I is

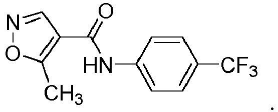

In a preferred embodiment, the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like;
preferably, the RNA virus is 2019 novel coronavirus (2019-nCoV), Ebola virus, avian influenza H9N2, H1N1 or H7N9.

In a preferred embodiment, the RNA virus includes, but not limited to: severe acute respiratory syndrome coronavirus (SARS-CoV), Middle East respiratory syndrome coronavirus (MERS-CoV) and 2019 novel coronavirus (2019-nCoV), Ebola virus, fever with thrombocytopenia syndrome virus (sftsv), avian influenza virus H9N2.

It should be understood that, within the scope of the present invention, each technical feature of the present invention described above and in the following (as examples) may be combined with each other to form a new or preferred technical solution, which is not listed herein due to the limitation on contents.

### Description of drawings

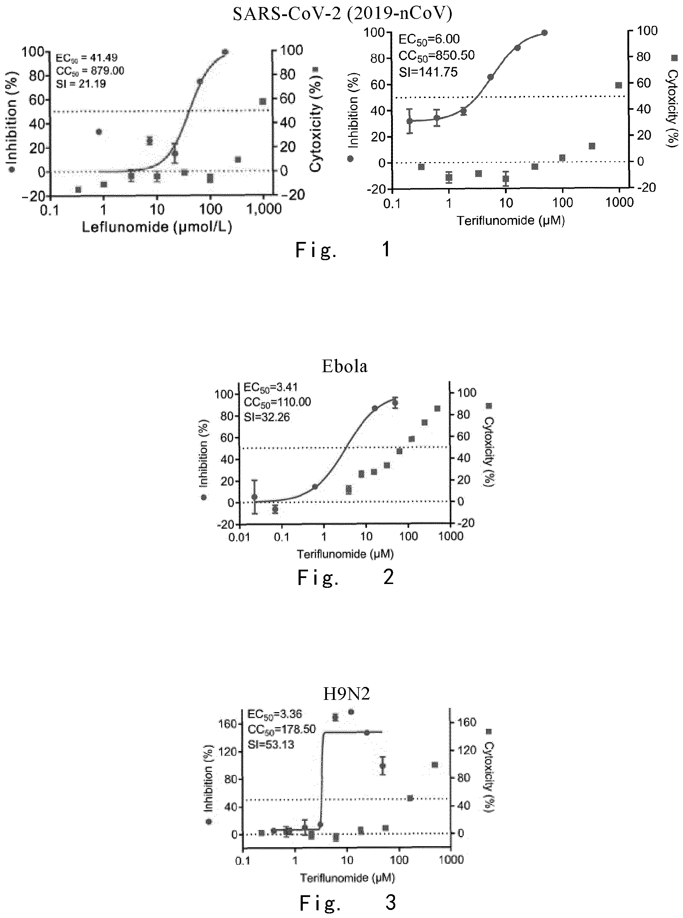

Figure 1 shows the inhibitory efficiency of teriflunomide and leflunomide against 2019-nCoV on Vero cells, respectively. The abscissa is the drug concentration, the ordinate is the inhibitory efficiency against the novel coronavirus, and the number of viral gene copies in the cell supernatant is used as the quantitative standard.
Figure 2 shows the inhibitory efficiency of teriflunomide against Ebola replicon on BSR T7/5 cells. The abscissa is the drug concentration, the ordinate is the inhibitory efficiency against Ebola virus replication, and the expression level of the reporter gene on the virus genome is used as a quantitative index.
Figure 3 shows the inhibitory efficiency of teriflunomide against H9N2 virus on MDCK cells. The abscissa is the drug concentration, the ordinate is the inhibitory efficiency against influenza virus replication, and the CPE degree of cell death caused by virus infection (quantitatively determined by Cell Titer-Glo) is used as a quantitative index.

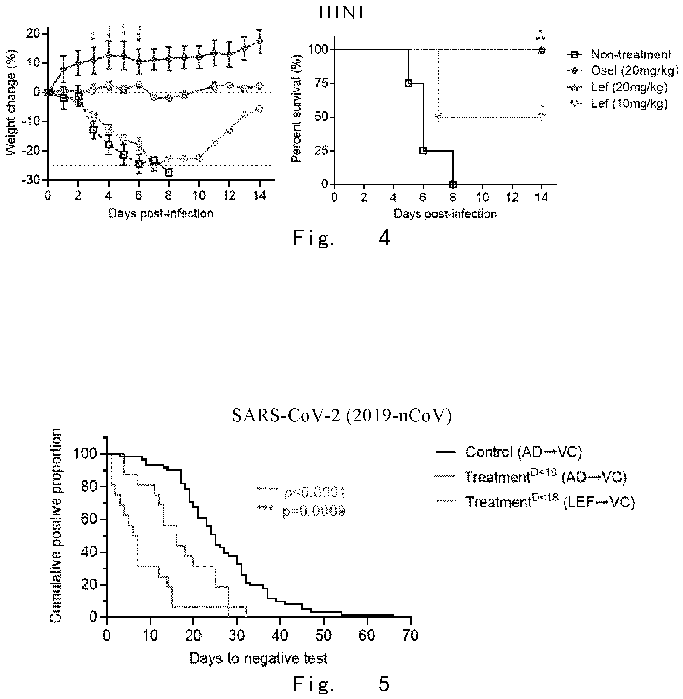

Figure 4 shows the antiviral efficacy of early administration of leflunomide in a mouse model of A/WSN/33(H1N1) influenza virus infection. The abscissa of the body weight curve on the left panel is the infection time, and the ordinate is the average percentage of changes in the body weight ± SEM. The abscissa of the survival curve on the right panel is the infection time, and the ordinate is the survival percentage. The black line represents Non-treatment group, the blue line represents marketed control drug Oseltamivir (Osel)-20 mg/kg group, and the green and red lines represent the leflunomide-10 and 20 mg/kg groups, respectively. Asterisks (^{∗}) indicate significance when the administration group is compared with the Non-treatment group. ^{∗}P < 0.05; ^{∗∗}P < 0.01; and ^{∗∗∗}P < 0.001.
Figure 5 shows therapeutic effects of leflunomide on compassionate use in patients with COVID-19. The abscissa is the time when the nucleic acid test turned negative, and the ordinate shows the percentage of the number of people who were positive in the nucleic acid test to the total number of people in this group. The black line represents the control group (the time from admission to negative conversion), the red line represents the time from admission to negative conversion in the leflunomide group within 18 days of admission, and the orange line represents the time from the start of taking leflunomide to negative conversion in the leflunomide group within 18 days of admission. Asterisks (^{∗}) indicate significance when the administration group is compared with the control group. ^{∗}P < 0.05; ^{∗∗}P < 0.01; ^{∗∗∗}P < 0.001 and ^{∗∗∗∗}P < 0.0001. AD, admission; LEF, taking leflunomide; VC, virus clearance.

### Modes for carrying out the invention

After extensive and in-depth research, the inventors found that the compounds of the present invention have broad-spectrum and excellent antiviral activities, especially significant inhibitory activities against the emerging 2019-nCoV, Ebola virus, bunya virus, avian influenza viruses, such as H9N2, while such compounds have lower toxicities. The present invention has been completed based on such findings.

### Definition

Scientific and technical terms used herein have the same meanings as understood by a skilled person in the art to which the present invention belongs. For clarity, some terms used herein are defined below.

As used herein, 2019 novel coronavirus, 2019-nCoV and SARS-CoV-2 have the same meaning.

As used herein, "alkyl" refers to a saturated branched or straight chain hydrocarbon group. Specifically, the term "alkyl" as used herein refers to a saturated branched or straight chain hydrocarbon group having 1-10 carbon atoms, preferably 2-8 carbon atoms, 1-6, 1-4 carbon atoms, 1-3 carbon atoms. Specific examples of alkyl include, but not limited to, methyl, ethyl, n-propyl, isopropyl, n-butyl, isobutyl, heptyl, and the like. As used herein, an alkyl may be substituted with one or more substituents, such as halogen or haloalkyl. For example, the alkyl may be an alkyl substituted with 1-4 fluorine atoms, such as trifluoromethyl, or the alkyl may be an alkyl substituted with a fluoroalkyl.

As used herein, "alkoxy" refers to a group as shown in RO-, i.e., a group attached to the rest of a molecule through an oxygen atom, wherein R is an alkyl as described above. In particular, the term "alkoxy" as used herein refers to an alkoxy having 1-10 carbon atoms, preferably 2-8 carbon atoms, 1-6, 1-4 carbon atoms, 1-3 carbon atoms, and the like. Specific examples of alkoxy include, but not limited to, methoxy, ethoxy, propoxy, butoxy, and the like. As used herein, an alkoxy may be substituted with one or more substituents, such as a halogen or haloalkyl. For example, the alkoxy may be an alkoxy substituted with 1-4 fluorine atoms, such as trifluoromethoxy, or the alkoxy may be an alkoxy substituted with a fluoroalkyl.

As used herein, "amino" refers to a group of formula "NRxRy", wherein Rx and Ry can be independently selected from H or a C1-C3 alkyl or C1-C3 haloalkyl. In a specific embodiment, "amino" as used herein refers to NH2.

As used herein, "halogen" refers to fluorine, chlorine, bromine or iodine. In a preferred embodiment, the halogen is chlorine or fluorine; more preferably fluorine.

As used herein, according to Article 23 of the Drug Administration Law of the People's Republic of China revised in August 2019, "compassionate use" refers to a medicament which is undergoing clinical trials for treating serious life-threatening diseases for which there is no effective treatment. If the medicament may be beneficial to patients through pharmaceutical observation and is in line with ethical principles, it can be used in other patients with the same condition in the institution where the clinical trial is conducted after review and informed consent. The compound used in the present invention, such as leflunomide, is a medicament that has been marketed, so that its dosage and safety can be well controlled.

### Compounds of the invention

As used herein, "the compound of the present invention" and "the compound of formula I" have the same meaning and can be used interchangeably.

As used herein, the inventors found that such compounds have broad-spectrum and excellent inhibitory activities against coronavirus, especially the newly emerging 2019-nCoV, Ebola virus, bunya virus, avian influenza viruses, such as H9N2. In a specific embodiment, the compound of the present invention is a compound of formula I or a pharmaceutically acceptable salt thereof: 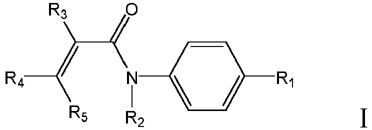 wherein R₁-R₅ are described as said above.

In particular, the inventors found that leflunomide, teriflunomide (A771726) have siginificant inhibitory activities against coronavirus, especially 2019-nCoV, Ebola virus, bunya virus, avian influenza viruses, such as H9N2.

Leflunomide is a new immunomodulatory drug approved by the U.S. Food and Drug Administration (FDA) in 1998 for the clinical treatment of rheumatoid arthritis, lupus erythematosus, a variety of primary and secondary glomerular disease, and prevention and treatment of graft rejection. Leflunomide is an isoxazole derivative with anti-proliferative activity, which can inhibit dihydroorotate synthase and directly inhibit the proliferation of lymphocytes and B cells by inhibiting the entire biosynthesis of pyrimidine. Teriflunomide is an oral inhibitor of pyrimidine synthase and immunomodulator, which is an active metabolite of leflunomide, can regulate immune function through various mechanisms, and exert anti-proliferative and anti-inflammatory effects. Therefore, leflunomide can be regarded as a prodrug of teriflunomide. However, the inventors found that, in addition to the known activities, leflunomide and teriflunomide showed excellent activities against coronavirus, Ebola virus, bunya virus, avian influenza virus; preferably activities against 2019-nCoV, Ebola virus, avian influenza H9N2, H1N1 or H7N9.

In addition, a skilled person can prepare the compounds of the present invention into various forms of prodrugs based on the common knowledge in the art and the contents of the present invention.

### Virus

The RNA virus described herein is a kind of biological virus, and their genetic material is composed of ribonucleic acid (RNA). Usually the nucleic acid is single-stranded (ssRNA single-stranded RNA), but also double-stranded ( dsRNA double-stranded RNA).

The term "coronaviruses" as used herein are single-stranded positive-stranded RNA viruses belonging to Orthocoronavirinae of Coronaviridae of Nidovirales. The virus can infect humans, bats, pigs, mice, cattle, horses, goats, monkeys and many other species. Seven coronaviruses (HCoVs) are known to infect humans, including Middle East respiratory syndrome-related coronavirus (MERSr-CoV) and severe acute respiratory syndrome-related coronavirus (SARSr-CoV).

The coronavirus isolated from the lower respiratory tract of patients with pneumonia of unknown cause in Wuhan is a new type of β-type coronavirus, named by WHO as 2019-nCoV, which is the 7^{th} coronavirus that can infect humans. At present, there are no effective vaccines and therapeutic drugs against the coronavirus, but mainly preventive measures to control the spread of the virus, closely monitor the epidemic situation, and isolate suspected cases for observation. At present, there is no effective treatment for coronavirus, and symptomatic and supportive treatment is mainly adopted.

Based on the above compounds, the present invention further provides a pharmaceutical composition for treating infections caused by viruses, especially RNA viruses, including but not limited to: coronaviruses, such as severe acute respiratory syndrome coronavirus (SARS-CoV), Middle East Respiratory Syndrome Coronavirus (MERSCoV) and 2019-nCoV, Ebola Virus, Bunya Virus, Avian Influenza H9N2, H1N1, H7N9, Arenavirus, Rabies Virus, Hepatitis C HCV, Type B Hepatitis HBV, human immunodeficiency virus HIV-1, herpes simplex virus HSV-1, HSV-2 and the like. The composition contains a therapeutically effective amount of a compound of the present invention, or a pharmaceutically acceptable salt thereof, and a pharmaceutically acceptable carrier or excipient. In a preferred embodiment, the compounds or pharmaceutical compositions of the present invention can be used for treating infections caused by severe acute respiratory syndrome coronavirus (SARS-CoV), Middle East respiratory syndrome coronavirus (MERS-CoV) and 2019-nCoV, Ebola virus, fever with thrombocytopenia syndrome virus (sftsv), avian influenza virus H9N2; preferably infections caused by 2019-nCoV, Ebola virus, avian influenza H9N2, H1N1 or H7N9.

Examples of pharmaceutically acceptable salts of the compounds of the present invention include, but not limited to, inorganic and organic acid salts, such as hydrochloride, hydrobromide, sulfate, citrate, lactate, tartrate, maleate , fumarate, mandelate, and oxalate; and inorganic and organic base salts with bases such as sodium hydroxy, tris(hydroxymethyl)aminomethane (TRIS, tromethamine) and N-methylglucamine.

Although each individual's needs vary, a skilled person can determine the optimal dosage of each active ingredient in the pharmaceutical compositions of the present invention. In general, a compound of the present invention, or a pharmaceutically acceptable salt thereof, is administered orally to mammals daily in an amount of about 0.0025 to 50 mg/kg body weight, preferably about 0.01 to 10 mg per kg. For example, a unit oral dosage may contain about 0.01 to 50 mg, preferably about 0.1 to 10 mg, of a compound of the present invention. A unit dosage may be administered one or more times, in one or more tablets per day, each tablet containing about 0.1 to 50 mg, preferably about 0.25 to 10 mg, of a compound of the present invention or a solvate thereof.

The pharmaceutical compositions of the present invention can be formulated into formulations suitable for various routes of administration, including but not limited to being formulated into a form for parenteral, subcutaneous, intravenous, intramuscular, intraperitoneal, transdermal, buccal, intrathecal, intracranial, nasal or topical administration for treating tumors and other diseases. The amount administered is an amount effective to ameliorate or eliminate one or more conditions. For treating a particular disease, an effective amount is an amount sufficient to ameliorate or in some way alleviate symptoms associated with the disease. Such amount can be administered as a single dose, or can be administered according to an effective therapeutic regimen. The amount administered may cure the disease, however the administration is usually to improve the symptoms of the disease. Repeated administration is generally required to achieve the desired symptomatic improvement. The dosage will be determined based on the patient's age, health and weight, the type of concurrent therapy, the frequency of therapy, and desired therapeutic benefits.

The pharmaceutical formulations of the present invention can be administered to any mammal so long as they can obtain the therapeutic effects of the compounds of the present invention. The most important of these mammals are humans. The compounds of the present invention or pharmaceutical compositions thereof can be used for treating ulcerative colitis.

The pharmaceutical formulations of the present invention can be manufactured in a known manner, for example, by conventional mixing, granulating, tableting, dissolving, or freeze-drying processes. In the manufacture of oral dosage forms, solid excipients and active compounds can be combined and the mixture can be optionally ground. After suitable auxiliaries, if desired or necessary, are added, the mixture of granules is processed to obtain tablets or dragee cores.

Suitable auxiliaries are especially fillers, for example sugars such as lactose or sucrose, mannitol or sorbitol; cellulose preparations or calcium phosphates, such as tricalcium phosphate or dicalcium phosphate; and binders, such as starch pastes, including corn starch , wheat starch, rice starch, potato starch, gelatin, tragacanth, methylcellulose, hydroxypropylmethylcellulose, sodium carboxymethylcellulose, or polyvinylpyrrolidone. If desired, disintegrants, such as the starches mentioned above, as well as carboxymethyl starch, cross-linked polyvinylpyrrolidone, agar, or alginic acid or a salt thereof, such as sodium alginate, can be added. Adjuvants are especially flow conditioners and lubricants, for example, silica, talc, stearates, such as magnesium calcium stearate, stearic acid or polyethylene glycols. If desired, dragee cores can be provided with a suitable coating resistant to gastric juices. For this purpose, concentrated sugar solutions can be used. Such solution may contain gum arabic, talc, polyvinylpyrrolidone, polyethylene glycol and/or titanium dioxide, lacquer solutions and suitable organic solvents or solvent mixtures. For preparing coatings resistant to gastric juices, suitable cellulose solutions such as cellulose acetate phthalate or hydroxypropyl methylcellulose phthalate can be used. Dyestuffs or pigments may be added to the coatings of tablets or dragee cores for, for example, the identification or characterization of combinations of active ingredients.

The administration method of the pharmaceutical composition includes but not limited to various administration methods well known in the art, and can be determined according to the actual situation of a patient. These methods include, but not limited to, parenteral, subcutaneous, intravenous, intramuscular, intraperitoneal, transdermal, buccal, intrathecal, intracranial, nasal, or topical routes of administration.

In addition to the compound of the present invention, the pharmaceutical composition of the present invention may also contain other antiviral drugs, and the other antiviral drugs may be selected from one or more of lopinavir, ritonavir, ribavirin, remdesivir, oseltamivir, Tamiflu, lanimivir and peramivir; preferably one or more of lopinavir, ritonavir, ribavirin, and remdesivir.

### Advantages of the invention

1. The present invention discovers for the first time a series of drugs having broad-spectrum and excellent antiviral activities, especially having signigficant inhibitory activities for the emerging 2019-nCoV, as well as Ebola virus, bunya virus, and avian influenza virus;
2. These drugs are less toxic to normal cells;
3. These drugs have laid the material foundation for the research and development of a new generation of antiviral drugs, and thus have important academic value and practical significance.

The present invention will be further described below with reference to specific examples. These examples are not intended to limit the scope of the invention. All the methods adopted according to the principles and technical means of the present invention belong to the scope of the present invention. In the following examples, the experiment method without specifying the specific conditions is usually based on the conventional conditions or the conditions recommended by the manufacturer. Percentages and parts are percentages and parts by weight, unless otherwise indicated.

### Example 1. Inhibitory activity and cytotoxicity evaluation of the compounds of the present invention on 2019-nCoV, Ebola virus, avian influenza virus A/GuangZhou/99 (H9N2)

Materials and Methods:
Both of leflunomide and teriflunomide are commercially available, with a purity of more than 98%.

### Detection methods and results:

### 1. Fluorescence quantitative PCR method to detect the efficacy of anti-2019-nCoV:

Vero E6 cells (ATCC-1586) were infected with 2019BetaCoV/Wuhan/WIV04/2019 strain (isolated by Wuhan Institute of Virology, Chinese Academy of Sciences, Zhou, P., et al. A pneumonia outbreak associated with a new coronavirus of probable bat origin. Nature 2020) at a MOI = 0.03 or MOI = 0.05, and at the same time, different dilution concentrations of drugs were added for co-culture. The drugs were diluted with DMSO and mixed with DMSO dilution served as a control. The infection solution was DMEM + 0.2% BSA. After 48 hours, the cell supernatant was collected, and the viral RNA in the supernatant was extracted by a viral RNA extraction kit (Qiagen), and the number of copies of viral RNA in the cell supernatant was detected by real-time quantitative reverse transcription PCR (qRT-PCR) (Qiagen) to reflect the replication efficiency of the virus. Data processing was performed by Graphpad prism software, and the half inhibitory concentration (IC₅₀) of the compound against virus was calculated.

### 2. Experiment to test the efficacy of anti-Ebola virus replicon by Bright-Glo

### Ebola virus replicon system (EBOV-NP, EBOV-VP35, EBOV-VP30, EBOV-MG, EBOV-L)

From the list of human-to-human pathogenic microorganisms, it can be seen that the hazard degree of Ebola virus is classified as the first category, which must be operated in a laboratory with a BSL-4 biosafety level. In order to reduce the risk of biosafety, Ebola virus replicon system was used to test the antiviral efficacy. This system can completely reflect the efficiency of Ebola virus replication and is a commonly used system for screening anti-Ebola virus drugs (Jasenosky L D, Neumann G, Kawaoka Y. Minigenome-Based Reporter System Suitable for High-Throughput Screening of Compounds Able to Inhibit Ebolavirus Replication and/or Transcription. Antimicrobial Agents & Chemotherapy. 2010. 54(7): 3007). The Ebola virus replicon system consists of a mini-genome expression plasmid MG recombinantly expressing T7 promoter and the luciferase gene, and 4 helper plasmids that express L, NP, VP35, and VP30 proteins, respectively. When the replicon system is transfected into a cell for replication, T7 RNA polymerase induces the initiation of T7 promoter, which in turn drives the expression of the luciferase gene (Jasenosky L D, Neumann G, Kawaoka Y. Minigenome-Based Reporter System Suitable for High-Throughput Screening of Compounds Able to Inhibit Ebolavirus Replication and/or Transcription. Antimicrobial Agents & Chemotherapy. 2010. 54(7): 3007). The replicon replication efficiency is positively correlated with the luciferase gene expression, therefore, the luciferase gene expression in the drug-treated cell system can be used to measure the replicon replication efficiency in the evaluation of a drug, so that the inhibition degree of the drug on Ebola replicon can be evaluated. Bright-Glo reagent (Promega) was used for the detection of luciferase expression. 2×10⁴ of BSR T7/5 cells (Generation of Bovine Respiratory Syncytial Virus (Brsv) from Cdna: Brsv Ns2 Is Not Essential for Virus Replication in Tissue Culture, and the Human Rsv Leader Region Acts as a Functional Brsv Genome Promoter. Journal of Virology. 1999. 73(1): 251-259, which is stably transfected and expresses T7 RNA polymerase gene, and the cell culture medium is MEM+10% FBS+1% L-Glutamine+2% MAA+1% P/S) were plated in a 96-well plate with a white bottom and used when the cells reached 80% confluence. The five-plasmid replication system for Ebola virus was prepared as follows:

| System | Concentration (ng) |
|---|---|
| EBOV-NP | 25 |
| EBOV-VP35 | 25 |
| EBOV-VP30 | 15 |
| EBOV-MG | 25 |
| EBOV-L | 150 |
| Total concentration | 240 |

Transfection: the plasmids of the above system were dissolved in 25 µL of Opti-MEM serum-free medium, marked as tube A; and 0.72 µL of Lipo 2000 was dissolved in 25 µL of Opti-MEM serum-free medium, marked as tube B. Tubes A and B were mixed and recorded as tube C, and placed at room temperature for 20 min. Compounds were added to the treated 96-well plate at 50 µL/well (the system without EBOV-L plasmid was used as the negative control). The plate was shaken at 800 rpm for 2 hours, the compounds were 3-fold diluted with Opti-MEM serum-free medium at 8 gradients, in triplicate, added to a shaken 96-well plate with white bottom at 50 µL per well, and placed in a 37°C, 5 %CO₂ incubator for 24 hours. And then, 50 µL of Bright-Glo reagent was added to each well, shaken for 3 min in the darkness, mixed well, and kept stand for 10 min. The plate was placed on a microplate reader for recording the Luminescence value, and the data were processed by Graphpad prism software to obtain the half inhibitory concentration of the compound on the virus IC₅₀.

### 3. Experiment for Detecting Drug Efficacy Against Human Infectious Avian Influenza Virus by Cell Titer-Glo

This experiment was based on the luminescence detection method of Cell Titer-Glo reagent, and the inhibitory activities of the compounds against the avian influenza virus A/GuangZhou/99 (H9N2) (the virus was donated by the National Influenza Center), which can infect humans, were detected. 2 × 10⁴ of MDCK cells were plated in a 96-well cell culture plate with a white bottom. After the cells grew into a monolayer, the original culture medium was discarded, and 50 µL of 20 TCID50 H9N2 avian influenza virus suspension and 50 µL of drug diluted solution were added at the same time, at least triplicate wells for each concentration. The virus infection solution is DMEM + 0.2% BSA + 25 mM HEPES + 1 µg/mL TPCK. A normal cell control group and a virus control group were also set. The 96-well cell culture plate was placed in a 37°C, 5% CO₂ incubator, and the CPE caused by the virus was observed under a microscope every day. The plate was removed from the incubator when 75%-100% CPE appeared in the virus control group. 25 µL of CellTiter-Glo^{®} reagent was added to each well, shaken for 3 min in the darkness, mixed well, and kept stand for 10 min. The plate was placed on a microplate reader for recording the Luminescence value, and the data were processed by Graphpad prism software to obtain the half inhibitory concentration of the compound on the virus IC₅₀.

### 4. Experiment to detect drug toxicity by CellTiter-Glo (CC₅₀)

Adenosine Tri-Phosphate (ATP) is involved in a variety of enzymatic reactions in organisms and is an indicator of the metabolism of living cells. The survival of cells can be detected by detecting the content of ATP in cells. In CellTiter-Glo live cell detection, fluorescence Luciferase is used as the detection substance. During the luminescence process, luciferase requires the participation of ATP. ATP can only be generated through the respiration of metabolically active cells and other life activities. An equal volume of CellTiter-Glo reagent was added to the cell culture medium, and the luminescence value was measured. The light signal is proportional to the amount of ATP in the system, and ATP is positively correlated with the number of living cells, so that the survival of the cells can be calculated. 2×10⁴ of cells were plated in a 96-well plate with a white bottom. After the cells grew into a monolayer, the original culture medium was discarded, and the compounds were diluted with infection medium (DMEM+0.2%BSA+25mM HEPES+1µg/mL TPCK) to different concentrations and added to a 96-well plate with 100 µl per well, 5 replicate wells for each concentration. The cells, into which only 0.1 mL of infection solution was added, were the normal control group. The plate was placed in a 37°C, 5% CO₂ incubator for 72 hours, afterwards taken out of the incubator and cooled to room temperature. 25 µL of CellTiter-Glo reagent was added to each well, shaken for 3 min in the darkness, mixed well, and kept stand for 10 min. The plate was placed on a microplate reader for recording the Luminescence value, and the data were processed by Graphpad prism software to obtain the semitoxic concentration (CC₅₀) and nontoxic limit concentration (MNCC) of compounds to cells. Cell viability % = Luminescence value of test well / Luminescence value of cell control well × 100%.

**Table 1. Antiviral efficacy of leflunomide and teriflunomide**

| Virus species | Teriflunomide | | Leflunomide | |
|---|---|---|---|---|
| | IC₅₀/CC₅₀ (µM) | SI 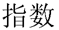 | IC₅₀/CC₅₀ (µM) | SI 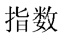 |
| 2019-nCoV | 6.001/850.5 | 141.75 | 41.49/879 | 21.19 |
| Ebola virus replicon Avian Influenza | 3.41/110 | 32.26 | 15.31/163.20 | 10.66 |
| A/GuangZhou/99 (H9N2) | 3.36/178.5 | 53.13 | 8.12/69.63 | 8.58 |

The IC₅₀ activity assays described above are as follows:
1. The inhibitory activity of teriflunomide against 2019-nCoV is that when the drug concentration is 6.001 µM = 1.62 µg/mL, the inhibitory efficiency on virus replication in Vero E6 cells can reach 50%, as shown in Figure 1.
2. The inhibitory activity of teriflunomide on Ebola replicon is that when the drug concentration is 3.41 µM = 0.923 µg/mL, the inhibitory efficiency on virus replication can reach 50%, as shown in Figure 2.
3. The inhibitory activity of teriflunomide on H9N2 avian influenza virus is that when the drug concentration is 3.36 µM = 0.9 µg/mL, the inhibitory efficiency on virus replication can reach 50%, as shown in Figure 3.

### discussion

It can be seen from the above results that teriflunomide exhibits good inhibitory activities against 2019-nCoV, Ebola virus, avian influenza virus A/GuangZhou/99 (H9N2), and excellent safety, therefore it has good application prospects. A skilled person can know that teriflunomide is an active metabolite of leflunomide, and for this reason, leflunomide can be regarded as a prodrug of teriflunomide to some extent. Therefore, although leflunomide exhibits slightly weaker inhibitory effects on 2019-nCoV, Ebola virus, and avian influenza virus A/GuangZhou/99 (H9N2), which is consistent with the effect as a prodrug, its *in vivo* may be the same as that of teriflunomide; that is, leflunomide also has excellent inhibitory effects on 2019-nCoV, Ebola virus, and avian influenza virus A/GuangZhou/99 (H9N2).

In view of the current global epidemic of 2019-nCoV, it is necessary to speed up the application research of leflunomide and teriflunomide.

### Example 2. Evaluation of antiviral activities of the compound of the present invention in combination with other antiviral drugs

The inventors further tested leflunomide, teriflunomide in combination with other antiviral drugs in the prior art, including one or more of lopinavir, ritonavir, ribavirin, remdesivir, oseltamivir, tamiflu, lanimivir, peramivir, and chloroquine (chloroquine phosphate).

It was found that leflunomide and teriflunomide used in combination with these antiviral drugs can produce better therapeutic effects, among which therapeutic effects obtained from the co-treatment with lopinavir, ritonavir, ribavirin, remdesivir and chloroquine (chloroquine phosphate) are relatively good.

### Example 3. Evaluation of anti-influenza virus efficacy of leflunomide in a mouse infection model

Experimental Materials:
Leflunomide is commercially available with a purity of more than 98%.

Detection method:
In this experiment, the anti-influenza efficacy of a drug was evaluated based on the mouse influenza virus A/WSN/33 (H1N1) infection model. The mice used were BALB/c female mice, 6-8 weeks old, weighing 18-22 g, and purchased from Beijing Weitong Lihua Laboratory Animal Technology Co., Ltd. All animal experiments were performed in the ABSL-2 laboratory. The mice were adapted to the ABSL-2 environment for 2-3 days before the experiment, and the mice were divided into following 4 groups: model group: Non-treatment; positive control group: Osel (oseltamivir) 20 mg/kg; and experimental group: Lef (leflunomide) 20 mg/kg and 10 mg/kg; 3 to 6 animals in each group. All mice were intranasally infected with A/WSN/33 (H1N1) 2LD₅₀ = 2000 pfu/mouse. The early administration was 3 hours before infection, and then once a day for 14 consecutive days. Body weight and survival data of mice were plotted to draw body weight change and survival rate curves using Graphpad prism software, and expressed as mean ± standard deviation (mean ± S.E.M.). Two-way ANOVA was used for statistical analysis. Compared with the control group, when P < 0.05, it was considered to be statistically significant.

Results:
The inventors further tested the efficacy of leflunomide against influenza virus in a mouse infection model. The drug was administered in the early stage of influenza virus infection in mice to verify the early anti-influenza efficacy of leflunomide. It was found that, in a mouse model of complete lethality, leflunomide admistered at 10 mg/kg rescued 50% of the mice and 20 mg/kg rescued 100% of the mice from death, as shown in Figure 4.

### Example 4. Therapeutic effects of leflunomide in compassionate use of Covid-19 patients

Experimental Materials:
Leflunomide is commercially available with a purity of more than 98%.

### Detection method:

### 1. Therapeutic effects of compassionate use in small-scale (10 cases) COVID-19 patients

From February 20, 2020 to February 28, 2020, 10 patients with laboratory-confirmed common COVID-19 patients with obvious pulmonary lesions were recruited from the Renmin Hospital of Wuhan University, five of whom orally received leflunomide, Leflunomide (10 mg/tablet), 50 mg/12 h, for three consecutive times followed by 20 mg/d for 10 days; the other five patients served as a blank control group without placebo. All patients received standard supportive care for COVID-19 (taking Arbidol, Lianhua Qingwen capsules, magnesium isoglycyrrhizinate, and cefoperazone). The patient's throat swab was sampled and the virus titer was detected by quantitative RT-PCR. Two consecutive negative results (Ct > 37) in the same patient were considered negative conversion. The negative conversion time and clinical detection indexes before and after treatment were compared between the control group and the experimental group.

### 2. Therapeutic effects of compassionate use in large-scale (132 cases) COVID-19 patients

From January 31, 2020 to April 5, 2020, 132 long-term positive COVID-19 patients and re-positive COVID-19 patients were recruited from the People's Hospital of Wuhan University. All patients received standard supportive treatment for COVID-19 (taking Arbidol, Lianhua Qingwen Capsules, Magnesium Isoglycyrrhizinate and Cefoperazone). In addition to basic treatment, patients in the experimental group were orally given leflunomide (10 mg/tablet) , 50 mg/12 h, followed by 20 mg/d for three consecutive times until discharge. In the final analysis, a total of 122 valid patient data were obtained, including 61 in the control group and 61 in the experimental group. 16 people in the experimental group received leflunomide within 18 days of admission. The patient's oral/pharyngeal swabs were sampled and the virus titer was detected by quantitative RT-PCR. Two consecutive negative results (Ct > 37) in the same patient were considered negative conversion. The negative conversion time and clinical detection indexes before and after treatment were compared between the control group and the experimental group.

Results:
The inventors further tested therapeutic effects of leflunomide of compassionate use in COVID-19 patients, and recruited COVID-19 patients to evaluate the effects of leflunomide in the treatment of COVID-19 patients. It was found that, in a small-scale (10 cases) clinical trial of compassionate use of COVID-19 patients, the time to negative conversion (median 5 days) of patients taking leflunomide was shorter than that of the control group (median 11 days, P = 0.046), and the level of C-reactive protein was also significantly reduced, indicating that leflunomide treatment can effectively control the immune pathological inflammation, as shown in Tables 2 and 3.

In a large-scale (132 cases) clinical trial of compassionate use of long-term positive COVID-19 patients and re-positive COVID-19 patients, the time from admission (AD) to negative conversion (VC) for COVID-19 patients who took leflunomide within 18 days of admission (median 16 days) was significantly shorter than that of the control group (median 25 days), and the time from taking leflunomide (LEF) to negative conversion (VC) in COVID-19 patients who took leflunomide within 18 days of admission (median 6.5 days) was significantly shorter than that of the control group, indicating that leflunomide treatment could significantly shorten the detoxification time of COVID-19 patients, as shown in Figure 5.

**Table 2. The time for patients to turn negative after treatment**

| Number of Patients | | leflunomide experimental group | | | control group | | | P value | |
|---|---|---|---|---|---|---|---|---|---|
| | | 1 | 3 | 4 | 1 | 4 | 5 | | |
| Time to negative conversion after treatment (d) | | 4 | 8 | 5 | 11 | 9 | 11 | 0.046 | |

| Table 3. C-reactive protein levels and differences thereof in patients before and after treatment | | | | | | | | | |
|---|---|---|---|---|---|---|---|---|---|
| parameter | leflunomide experimental group, n = 5 | | | | control group, n = 5 | | | | |
| | before treatment | after treatment | | P value | before treatment | | after treatment | | P value |
| CRP, mg/L (0-10) | 37.4 (7.8-120.6) | 5 (5-5) | | 0.109 | 5 (5-14.75) | | 5.58 (3.27-7.47) | | 0.893 |

## Claims

1. Use of a compound of formula I, or a pharmaceutically acceptable salt thereof, in the preparation of a drug against RNA virus infection: 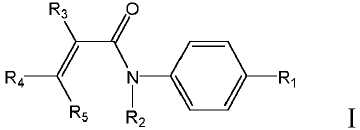 wherein, R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.

2. The use of claim 1, wherein in formula I,
R₁ is selected from: H, a substituted or unsubstituted C1-3 alkyl, substituted or unsubstituted C1-3 alkoxy;
R₂ is selected from: H, a substituted or unsubstituted C1-3 alkyl;
R₃ is selected from: H, a cyano, substituted or unsubstituted C1-3 alkyl, substituted or unsubstituted C1-3 alkoxy;
R₄ is selected from: H, a hydroxyl, substituted or unsubstituted C1-3 alkyl, substituted or unsubstituted C1-3 alkoxy; alternatively, R₃ and R₄ may be joined to form a 5-6 membered ring containing 2 heteroatoms selected from N, O or S;
R₅ is selected from: H, a substituted or unsubstituted C1-3 alkyl, substituted or unsubstituted C1-3 alkoxy.

3. The use of claim 1, wherein the compound of formula I is a compound selected from the group consisting of: 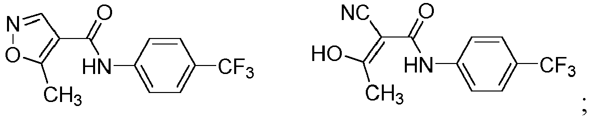 preferably, the compound of formula I is

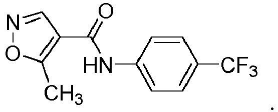

4. The use of any one of claims 1-3, wherein the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like;
preferably, the RNA virus is 2019 novel coronavirus (2019-nCoV), Ebola virus, avian influenza H9N2, H1N1 or H7N9.

5. A pharmaceutical composition comprising a compound of formula I, or a pharmaceutically acceptable salt thereof and other antiviral drugs, 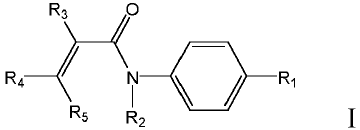 wherein R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.

6. The pharmaceutical composition of claim 5, wherein the compound of formula I is a compound selected from the group consisting of: 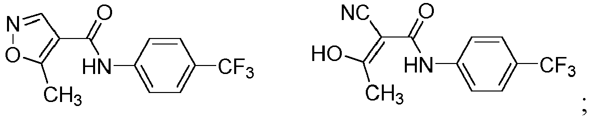 preferably, the compound of formula I is

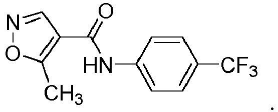

7. The pharmaceutical composition of claim 5 or 6, wherein the other antiviral drugs include, but not limited to: one or more of lopinavir, ritonavir, ribavirin, remdesivir, oseltamivir, Tamiflu, lanimil, weperamivir, arbidol and chloroquine (chloroquine phosphate) and the like; and preferably one or more of lopinavir, ritonavir, ribavirin, remdesivir and chloroquine (chloroquine phosphate).

8. A method for treating RNA virus infection, comprising administering a therapeutically effective amount of a compound of formula I or a pharmaceutically acceptable salt thereof or the pharmaceutical composition described in the second aspect to a subject in need of the treatment for viral infections: 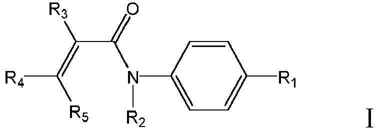 wherein R₁ is selected from: H, a substituted or unsubstituted C1-6 alkyl, substituted or unsubstituted C1-6 alkoxy, halogen, nitro, hydroxyl, cyano, amino;
R₂ is selected from: H, a substituted or unsubstituted C1-6 alkyl;
R₃ is selected from: H, a cyano, substituted or unsubstituted C1-6 alkyl, substituted or unsubstituted C1-6 alkoxy, halogen, nitro, hydroxyl, amino;
R₄ is selected from: H, a hydroxyl, cyano, substituted or unsubstituted C1-6 alkyl, substituted or unsubstituted C1-6 alkoxy, halogen, nitro, amino; alternatively, R₃ and R₄ may be joined to form a 5-7 membered ring containing 1-3 heteroatoms selected from N, O or S;
R₅ is selected from: H, a substituted or unsubstituted C1-6 alkyl, substituted or unsubstituted C1-6 alkoxy, halogen, nitro, hydroxyl, cyano, amino.

9. The method of claim 8, wherein the compound of formula I is a compound selected from the group consisting of: 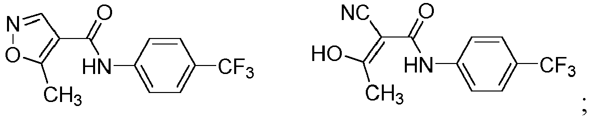 preferably, the compound of formula I is

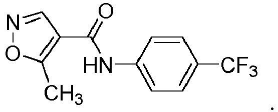

10. The method of claim 8 or 9, wherein the RNA virus includes, but not limited to, coronaviruses, such as severe acute respiratory syndrome coronavirus (SARS-CoV), Middle East respiratory syndrome coronavirus (MERSCoV) and 2019 novel coronavirus (2019-nCoV), Ebola virus, bunya virus, avian influenza H9N2, H1N1, H7N9, arena virus, rabies virus, hepatitis C HCV, hepatitis B HBV, human immunodeficiency virus HIV-1, herpes simplex virus HSV-1, HSV-2, and the like;
preferably, the RNA virus is 2019 novel coronavirus (2019-nCoV), Ebola virus, avian influenza H9N2, H1N1 or H7N9.
